# EUROPÄISCHE PATENTSCHRIFT

(11) **EP 1 368 286 B1**
(45) Veröffentlichungstag und Bekanntmachung des Hinweises auf die Patenterteilung: **02.12.2009**
(21) Anmeldenummer: 02712940.2
(22) Anmeldetag: 08.03.2002
(51) Int. Cl.: C04B 38/00, C04B 38/06, C04B 41/83, A61K 6/06, A61K 6/083

(54) **VERBUNDWERKSTOFF UND VERFAHREN ZU SEINER HERSTELLUNG**
COMPOSITE MATERIAL AND METHOD FOR THE PRODUCTION THEREOF
MATERIAU COMPOSITE ET SON PROCEDE DE PRODUCTION

(30) Priorität: 10.03.2001 EP 01105991
(43) Veröffentlichungstag der Anmeldung: 10.12.2003
(73) Patentinhaber: Vita Zahnfabrik H. Rauter GmbH & Co. KG, D-79704 Bad Säckingen (DE)
(72) Erfinder: AECHTNER, Stefan, 79713 Bad Säckingen (DE); HORNBERGER, Helga, 90425 Nürenberg (DE); NAGEL, Emil, 79713 Bad Säckingen (DE); THIEL, Norbert, 79713 Bad Säckingen (DE)
(74) Vertreter: Meyers, Hans-Wilhelm
(86) Internationale Anmeldenummer: PCT/EP2002/002567
(87) Internationale Veröffentlichungsnummer: WO 2002/076907

(56) Entgegenhaltungen:
- EP-A- 0 701 808
- EP-A- 0 803 241
- EP-A- 1 006 095

## Beschreibung

Die vorliegende Erfindung betrifft einen Verbundwerkstoff mit einem porösen anorganisch-nichtmetallischen Netzwerk, dessen Poren mit einem Polymer gefüllt sind, ein Verfahren zu seiner Herstellung, sowie Verwendungen dieses Verbundwerkstoffs.

Die EP-A-1 006 095 betrifft ein Verfahren zur Herstellung endodontischer, orthodontischer und direkter Restaurationen aufbauend auf einem keramischen Netzwerk. Ausgehend von einem Glasmonomer, wird dieses mit einem Material gefüllt, um verbesserte Flexibilität und Abriebbeständigkeit zu erreichen. Das Infiltrationsmaterial kann beispielsweise ein Glas sein, das dann eine Schicht bildet mit einer Härte im Bereich von 300 - 600 KHN und einem Elastizitätsmodul im Bereich von 70 - 80 GPa aufweist. Die Biegebruchfestigkeit liegt im Bereich von 200 - 500 MPa. Im Inneren soll die Struktur eine Biegebruchfestigkeit von etwa 150 - 80 MPa und einen Elastizitätsmodul von 15 - 25 GPa aufweisen. Entsprechende Werte sollen durch Infiltration mit einem Monomer und anschließender Härtung erreicht werden. Damit ist ein zweischichtiger Aufbau beschrieben, der unterschiedliche Eigenschaften aufweist. Entsprechende Produkte sind bislang nicht bekannt.

Die EP-A-0 803 241 betrifft ein Dentalmaterial aus einer porösen Keramik, die in einen künstlichen Zahn gefüllt wird, ein Inlay, ein Onlay, eine Krone, eine Kronenbrücke oder in einem Block, der geeignet für CAD/CAM-Verarbeitung ist. Dieses Material wird gesintert, wobei miteinander in Verbindung stehende Poren entstehen. Anschließend wird mit einem Kunstharz imprägniert. Nachteilig an diesen beschriebenen Produkten ist die nicht genaue Definition und Beschreibung des zugrundeliegenden Netzwerks. Dies führt in vielen Ausführungen zur Verschlechterung der physikalisch-technischen Daten im Vergleich zu heute gängigen Kompositmaterialien. Die US-A-5,843,348 betrifft ein Verfahren zur Herstellung eines keramischen Netzwerkmaterials aus einer keramischen Suspension. Dieses Verfahren beinhaltet das Gießen einer Suspension in eine Form, die zahnförmig ausgestaltet ist. Die Suspension enthält dispergierte Aluminiumoxidpartikel in einem Medium, das deionisiertes Wasser mit einem pH im Bereich von 4 - 5 und Polyvinylalkohol in einer Konzentration von 0,5 - 1 Gew.-% enthält. Nach Trocknung der Suspension wird diese in einem Ofen bei einer Temperatur im Bereich von 1000 - 1400°C gebrannt, um ein keramisches Netzwerk zu erzeugen. Die poröse Struktur wird mit Lanthanaluminosilikatglas infundiert, so dass eine Glasschicht mit einer Dicke in einem Bereich von 1 - 2 mm innerhalb des keramischen Netzwerkes entsteht.

In Ullmanns Encyclopedia of Industrial Chemistry werden verschiedene Verfahren zur Konservierung von porösen Steinstrukturen beschrieben. Dabei werden die Objekte, z.B. Skulpturen aus Sandstein oder Marmor, mit hydrophoben Reagenzien wie Alkyltrialkoxysilanen und/oder Kopplungsreagenzien wie Tetraethyorthosilikat imprägniert. Zur Stabilisierung der Struktur werden die Poren außerdem unter Vakuum mit Monomeren, beispielsweise Methylmethacrylat, infiltriert. Nach der thermischen Aushärtung unter Druck sind die Objekte mechanisch stabiler und vor allem ist dem chemischen Zerfall durch den Verschluss der Poren weitgehend Einhalt geboten.

Der Erfindung liegt die Aufgabe zu Grunde, einen Verbundwerkstoff zu schaffen, der ein homogenes porenfreies Gefüge und Eigenschaften aufweist, die zwischen keramischen und konventionell gefüllten Polymerwerkstoffen liegen, und ein Verfahren zur Herstellung dieses Verbundwerkstoffs aufzuzeigen.

Diese Aufgabe wird gelöst durch einen Verbundwerkstoff mit einer porösen anorganisch nichtmetallischen Matrix und einem zweiten Material, **dadurch gekennzeichnet, dass**
- die poröse anorganische nichtmetallische Matrix eine Biegebruchfestigkeit von ≥ 40 Mpa, gemessen nach ISO 6 872, besitzt,
- das zweite Material ein organisches Material ist, das die Poren der porösen Matrix mindestens teilweise ausfüllt und der Verbundwerkstoff einen Elastizitätsmodul E ≥ 25 GPa gemessen nach ISO 10 477 aufweist.

Der erfindungsgemäße Verbundwerkstoff ist vorteilhaft, weil eine neue Werkstoffklasse erreicht wird, deren Eigenschaften zwischen Keramik- und Kunststoffmaterialien liegen. Zum Beispiel zeichnet sich diese Werkstoffklasse einerseits durch eine geringere Sprödigkeit als Keramik und andererseits durch eine erhöhte Abriebfestigkeit gegenüber den bisherigen anorganisch gefüllten Polymeren aus.

Die anorganisch-nichtmetallische Phase des erfindungsgemäßen Verbundwerkstoffs bildet ein Netzwerk, das eine Eigenfestigkeit (Biegebruchfestigkeit des porösen anorganisch-nichtmetallischen Netzwerks vor Infiltration) von σ ≥ 40 MPa besitzt. Dadurch findet eine Erhöhung des Elastizitätsmodul des Verbundwerkstoffes im Vergleich zu herkömmlich gefüllten Kompositen statt.

Der erfindungsgemäße Verbundwerkstoff weist vorzugsweise eine Biegebruchfestigkeit von σ ≥ 100 Mpa, gemessen nach ISO 6 872 auf.

Die Poren des porösen Netzwerks nehmen ein Volumen von 5 % bis 85 Vol%, vorzugsweise 10 bis 50 Vol%, insbesondere 15 bis 35 Vol% ein. Ein Vorteil einer einstellbaren Porosität des anorganisch-nichtmetallischen Netzwerks ist der damit korrelierende Polymeranteil im späteren Verbundwerkstoff. Die Erhöhung des Polymeranteils ist verbunden mit einer Erhöhung der Schlagzähigkeit und der Abnahme des spezifischen Gewichts des Verbundwerkstoffs.

Typischerweise weisen die Poren des anorganisch-nichtmetallischen Netzwerks des erfindungsgemäßen Verbundwerkstoffs eine Porengröße von 0,2 bis 25 µm, vorzugsweise 0,5 bis 10 µm, auf. Die zu verwendenden Porengrößen und -formen sind abhängig von der Werkstoffkombination aus Anorganik und Polymer, vom Benetzungswinkel zwischen Anorganik und Monomer bzw. Polymer, von der Vorbehandlung und vom verwendeten Infiltrationsverfahren.

Das poröse anorganisch-nichtmetallische Netzwerk als Zwischenschritt zum erfindungsgemäßen Verbundwerkstoff ist beispielsweise durch Sinterung von pulverförmigen anorganischen, insbesondere keramischen Substanzen oder Substanzmischungen erhältlich, wobei der Sinterungsprozess insbesondere vor der Bildung geschlossener Poren beendet wird. Bevorzugt wird weiterhin, dass der Sinterprozess erst nach der Phase der Sinterhalsbildung oder des Glasflusses beendet wird, die typischerweise mit einer sprunghaften Änderung der Eigenfestigkeit des anorganisch-nichtmetallischen Netzwerkes verbunden ist.

Vorzugsweise werden pulverförmige anorganische Substanzen oder Substanzmischungen mit Korngrößen eingesetzt, die eine bimodale Verteilung, z.B. feine und grobe Korngrößen, aufweisen. Dabei weisen die feinen Körner eine höhere Sinteraktivität auf, die großen Körner bestimmen die Porenform.

Das anorganisch-nichtmetallische Netzwerk des erfindungsgemäßen Verbundwerkstoffs weist in einer bevorzugten Ausführungsform pulverförmige anorganische Substanzen oder Substanzmischungen auf, die eine Korngröße von 0,2. µm bis 25 µm besitzen. Typische d₅₀ Werte (Lasergranulometrie) der verwendeten Ausgangsmaterialien liegen zwischen 0,5 und 5 µm.

Das anorganisch-nichtmetallische Netzwerk des erfindungsgemäßen Verbundwerkstoffs ist vorzugsweise aus mindestens zwei verschiedenen Pulvermischungen mit unterschiedlichen Sintertemperaturen aufgebaut worden. Die Sinteraktivität wird durch die niedrigschmelzenden Pulverkomponenten bestimmt.

Die Poren des anorganisch-nichtmetallischen Netzwerks weisen vorteilhafterweise Oberflächen auf, die hydrophobe Eigenschaften besitzen. Die hydrophopen Eigenschaften können zum Beispiel durch oberflächliche Silanisierung erreicht werden. Die hydrophoben Oberflächen erhöhen die Benetzbarkeit des porösen Netzwerks mit Monomeren.

Die Silanisierung erfolgt in einfacher Weise mittels eines Silanisierungmittels in flüssiger Phase.

Zur Silanisierung des porösen anorganisch-nichtmetallischen Netzwerks wird ein Alkoxysilan oder ein Halogensilan, vorzugsweise 3-Methacryloxypropyl-trimethoxysilan, eingesetzt.

Die organische Phase des erfindungsgemäßen Verbundwerkstoffs ist insbesondere ein organisches Polymer, das durch Präpolymere, Oligomere oder Monomere in situ in den Poren des porösen Netzwerks durch Polymerisation gebildet wird. Das organische Polymer wird aus thermisch polymerisierbaren Monomeren und/oder durch chemisch induzierte Starterreaktionen polymerisierbarer Monomere und/oder durch kondensierbare Monomere gebildet.

Der erfindungsgemäße Verbundwerkstoff ist vorzugsweise isotrop. Dadurch entfallen die Nachteile der heutigen anisotropen Komposite, die z.B. bei Beanspruchung gegen die Vorzugsrichtung nur geringe Festigkeiten aufweisen. Dennoch ist es vorstellbar, dass eine gezielte Anisotropie in den erfindungsgemäßen Verbundwerkstoff eingebracht werden kann. Dies kann z.B. durch Einbringen von Fasern, die in andere Raumrichtungen gezielt vernetzt werden, erfolgen. Des weiteren ist durch Verwendung von Gradientenöfen zur Herstellung des anorganisch-nichtmetallischen Netzwerks, die Herstellung von kontinuierlichen Gradientenwerkstoffen möglich.

Der erfindungsgemäße Verbundwerkstoff kann Hilfsstoffe, wie Antioxidantien und für den jeweiligen Anwendungszweck geeignete Pigmente aufweisen.

Der erfindungsgemäße Verbundwerkstoff kann z. B. durch ein Verfahren wie folgt hergestellt werden:
- Herstellen des anorganisch-nichtmetallischen Ausgangsmaterials,
- Formgebungsverfahren der anorganisch-nichtmetallischen Phase, nass z.B. Schlickern oder trocken z. B. isostatisches Pressen, gegebenenfalls unter Verwendung eines geeigneten Bindersystems.
- Sintern des anorganisch-nichtmetallischen Netzwerks auf den gewünschten Sintergrad und Porosität,
- wobei der Sinterprozess beendet wird, bevor im wesentlichen geschlossene Poren im Sinterprodukt entstehen,
- und nachdem die Phase der Sinterhalsbildung und/oder Glasflusses erreicht worden ist,
- das erhaltene poröse Produkt aus anorganisch-nichtmetallischen Material wird zunächst mit einem Benetzungsmittel, bevorzugt Silane mit einer geeigneten funktionellen Gruppe, belegt,
- dann wird das anorganisch-nichtmetallische Netzwerk vollständig mit Monomeren infiltriert,
- und anschließend mit einem geeigneten Verfahren, wie z.B. Heißpolymerisation oder Mikrowellen, polymerisiert.

Im erfindungsgemäßen Verfahren werden als anorganisches Material z.B. Oxidkeramiken, Gläser, Porzellane, Nichtoxidkeramiken und Kombinationen derselben eingesetzt.

Im erfindungsgemäßen Verfahren werden insbesondere pulverförmige anorganische Materialien mit einer Korngröße von 0,2 µm bis 25 µm, vorzugsweise 0,5 bis 10 µm (d₅₀-Werte bestimmt mittels Lasergranulometrie), eingesetzt.

Vorzugsweise wird erfindungsgemäß das organische Material in flüssiger Form in das gesinterte anorganische Material infiltriert.

Das Benetzungsmittel liegt vorzugsweise in Lösung vor. Ein Vorteil der Verdünnung liegt in der erniedrigten Viskosität.

Das Benetzungsmittel muss eine kopplungsfähige funktionelle Gruppe enthalten.

Erfindungsgemäß werden organische Monomere oder Präpolymere in das gesinterte anorganisch-nichtmetallische Netzwerk eingebracht und in den Poren des Netzwerks zu dem organischen Material polymerisiert.

Das organische Material kann erfindungsgemäß durch Druckinfiltration in das gesinterte anorganische Material eingebracht werden. Der Vorteil besteht in der schnellen vollständigen und homogenen Durchdringung. Es kann sich je nach Aufgabenstellung als Vorteil erweisen, die Polymerisation unter Druck durchzuführen.

Gegebenenfalls sind vor der Infiltration sowohl das anorganisch-nichtmetallische Netzwerk als auch das organische Monomer zu evakuieren.

Als Monomere werden dabei vorzugsweise organische Verbindungen eingesetzt, die mindestens eine ethylenisch ungesättigte Struktureinheit, mindestens eine kondensierbare Struktureinheit oder mindestens eine ringöffnende polymerisierbare Struktureinheit oder Kombinationen derselben aufweisen.

Geeignete Startersysteme für die Polymerisation sind bekannt und der entsprechenden Literatur zu entnehmen.

Zur Herstellung transluzenter für Dentalzwecke geeigneter Materialien werden erfindungsgemäß feldspathaltige Pulver und Fritten als anorganisches Material und Bismethacrylate als organische Verbindung sowie peroxidhaltige Verbindungen als Starter eingesetzt.

Gegenstand der Erfindung ist auch ein Verbundwerkstoff für dentale Anwendungen, der durch das erfindungsgemäße Verfahren erhältlich ist.

Der erfindungsgemäße Verbundwerkstoff kann bevorzugt für dentale Zwecke, wie z.B. für Inlays, Onlays, Kronen und Brücken verwendet werden.

Erfindungsgemäß hergestellte Verbundwerkstoffe sind u.a. verwendbar zur Oberflächenvergütung von keramischen und metallischen Werkstoffen, Kompositen und Kunststoffen, als Bauteile mit neuartigen Eigenschaften wie z.B. Elastizitätsmodul, Abrasionsverhalten, spez. Gewicht, Formstabilität bei erhöhten Temperaturen. Weitere Ausführungen sind schalldämmende und wärmedämmende Elemente, Gleitlager, Elemente zur Vibrationsdämpfung, elektrische Isolatoren usw.

Insbesondere kann der erfindungsgemäße Verbundwerkstoff als Gleitlager, zur Wärme- und/oder Schalldämmung, oder als Vibrationsdämpfer eingesetzt werden.

Die zur Herstellung des erfindungsgemäßen Verbundwerkstoffes einsetzbare poröse anorganische nichtmetallische Matrix mit einer Biegebruchfestigkeit von ≥ 40 Mpa, gemessen nach ISO 6 872 kann als Zwischenprodukt dienen.

Vorzugsweise wird die poröse anorganische nichtmetallische Matrix aus Oxidkeramik, Gläsern, Porzellanen, Nichtoxidkeramiken oder Kombinationen davon gebildet.

Die Poren der porösen anorganischen nichtmetallischen Matrix nehmen insbesondere ein Volumen von 5 Vol % bis 85 Vol %, vorzugsweise 10 bis 50 Vol %, insbesondere 15 - 35 Vol % ein.

Ein Verfahren zur Herstellung einer erfindungsgemäßen porösen anorganisch-nichtmetallischen Matrix weist die Schritte auf :
- wobei ein anorganisches nichtmetallisches Material mit einem entfernbaren Binder zu einem formbaren Material gemischt wird,
- der Binder entfernt wird unter Erhalt einer porösen anorganischen nichtmetallischen Struktur,
- die poröse anorganische nichtmetallische Struktur gesintert wird,
- unter Ausbildung der porösen anorganisch- nichtmetallischen Matrix.

Ein Verfahren zur Abformung von Gegenständen, unter Verwendung der erfindungsgemäßen porösen anorganisch-nichtmetallischen Matrix ist **dadurch gekennzeichnet, dass**
- ein anorganisches nichtmetallisches Material mit einem entfernbaren Binder zu einem formbaren Material gemischt wird,
- das formbare Material mit einem abzuformenden Gegenstand in Kontakt gebracht wird, so dass der abzuformende Gegenstand in Negativform im formbaren Material abgeformt wird,
- das formbare Material unter Erhalt der Form des abgeformten Gegenstandes abgelöst wird und dann der Binder entfernt wird,
- danach gegebenenfalls die nach dem Entfernen des Binders erhaltene Struktur gesintert und infiltriert wird.

In einer Weiterbildung des genannten Verfahrens wird ein Verfahren zur Replikation von Gegenständen erhalten. Dabei wird zunächst das Verfahren zur Abformung von Gegenständen durchgeführt. Die dadurch erhaltene Matrix wird selbst abgeformt, unter Erhalt eines Replikates des zu replizierenden Gegenstands. Es kann aber auch von einer auf andere Weise gewonnenen Abformung eines Gegenstandes ausgegangen werden, unter Erhalt einer Negativform, die dann gemäß des erfindungsgemäßen Abformverfahrens abgeformt und gegebenenfalls verfestigt wird.

Einsetzbar ist auch eine Mischung aus pulverförmigen anorganischen nichtmetallischen Substanzen oder Substanzmischungen und entfernbarem Binder. Diese Mischung ist dadurch gekennzeichnet, dass die pulverförmigen anorganischen nichtmetallischen Substanzen oder Substanzmischungen eine Korngröße d₅₀ von 0,2 µm bis 25 µm aufweisen und der Binder in einer Menge von 2 Gew % bis 50 Gew %, bezogen auf das Gesamtgewicht der Mischung, vorliegt.

Die pulverförmigen anorganischen nichtmetallischen Substanzen oder Substanzmischungen sind vorzugsweise Oxidkeramik, Gläser, Porzellane, Nichtoxidkeramiken oder Kombinationen davon.

Vorzugsweise werden pulverförmige anorganische Substanzen oder Substanzmischungen mit Korngrößen eingesetzt, die eine bimodale Verteilung aufweisen.

Die pulverförmigen anorganischen nichtmetallischen Substanzen oder Substanzmischungen zur Herstellung der erfindungsgemäßen Paste weisen insbesondere eine Korngröße d₅₀ von 0,5 bis 5 µm, gemessen mittels Lasergranulometrie, auf.

In der Mischung zur Herstellung der Paste können mindestens zwei verschiedene Pulver und/oder Pulvermischungen mit unterschiedlichen Sintertemperaturen vorliegen. In der erfindungsgemäßen Paste sind Zusätze enthalten, die ein gezieltes Aushärten nach Stand der Technik erlauben.

Mittels der porösen anorganisch nichtmetallischen Matrix lassen sich Formkörper erhalten, die vorteilhafte Eigenschaften besitzen und auch Gegenstand der Erfindung sind. Die erfindungsgemäßen Formkörper sind aus porös gesinterten natürlichen und/oder synthetischen Feldspäten oder Feldspatoiden mit einer Porosität entsprechend einem Volumen von 5 Vol % bis 85 Vol %, vorzugsweise 10 bis 50 Vol %, insbesondere 15 - 35 Vol % herstellbar. Durch Infiltration mit geeigneten Monomeren und anschließender Polymerisation sind transparente, für dentale Anwendung (z.B. Verwendung als Inlays, Onlays, Veneers Kronen oder Brücken) geeignete Werkstoffe herstellbar.

Insbesondere vorteilhaft sind mehrschichtige Formkörper mit mehreren Schichten mit unterschiedlichen Eigenschaften in den Schichten erhältlich durch Auftragen verschiedener Pasten mit unterschiedlichen resultierenden anorganisch-nichtmetallischen Matrixkomponenten, anschließendem Sintern D Die Herstellung des kompletten Verbundwerkstoffs erfolgt dann wie bereits beschrieben.

Auch Formkörper mit kontinuierlich ändernden Eigenschaften sind gemäß der Erfindung herstellbar und Gegenstand der Erfindung. Diese Formkörper sind erhältlich durch z.B. Sintern der anorganisch-nichtmetallischen Matrix in einem Gradientenofen. Auch der natürliche Zahn ist anisotrop und aus mehreren unterschiedlichen Schichten aufgebaut Typischerweise werden die erfindungsgemäßen Formkörper wie folgt hergestellt
- Präparation der Arbeit im Mund, Aufbringen eines Trennmittels
- bei Kavitäten direkte Abformung im Mund mittels der erfindungsgemäßen Paste
- Härtung oder Aushärtung des Formkörpers, Entfernen aus der Kavität
- Sintern auf Porosität unter Ausbrennen des Binders.
- bei Kronen und Brücken: Abformung der Präparation
- Erstellen des Meistermodells
- Aufbau der Restauration auf dem Modell mittels erfindungsgemäßer Paste
- Sintern der Arbeit auf Porosität unter Ausbrennen des Binders.

Die Erfindung wird anhand der folgenden Beispiele näher erläutert.

### Beispiel 1:

Ein bimodales Aluminiumoxid mit einem d-50 von ca. 2,5 µm wurde mit destilliertem Wasser und üblichen Zusätzen (hier Citronensäure und Darvan) sowie Ultraschall zu einer schlicker-fähigen Suspension angerührt. Mit dieser Suspension wurden Delrinformen mit den Maßen 1,2x4x20mm ausgegossen. Nach Trocknung wurden die Teile entformt und bei einer Spitzentemperatur von 1120 °C und einer Haltezeit von 2 Stunden gebrannt.

Die gebrannten porösen Teile wurden dann mit einer 5%igen Lösung von Methacryloxypropyl-Trimethoxysilan getränkt und erneut getrocknet. Danach wurden die Teile evakuiert und mit einer evakuierten 1:1-Mischung aus Urethandimethacrylat und Triethylenglykoldimethacrylat über Nacht bei Raumtemperatur infiltriert und anschließend über einen Zeitraum von 17 Std. polymerisiert , wobei die Spitzentemperatur 80 °C betrug.

Die Teile zeigten auf einer Universalprüfmaschine der Fa. Zwick eine mittlere Biegefestigkeit von 300,15 MPa und einen Elastizitätsmodul von 76,23 GPa.

### Beispiel 2:

Ein bimodales Magnesium-Aluminiumoxid-Spinell mit einem d-50 von ca. 2,5µm wurde mit destilliertem Wasser und üblichen Zusätzen (hier Citronensäure und Darvan) sowie Ultraschall zu einer schlickerfähigen Suspension angerührt. Mit dieser Suspension wurden Delrinformen mit den Maßen 1,2x4x20mm ausgegossen. Nach Trocknung wurden die Teile entformt und bei einer Spitzentemperatur von 1180 °C und einer Haltezeit von 2 Stunden gebrannt.

Die gebrannten porösen Teile wurden dann mit einer 5%igen Lösung von Methacryloxypropyl-Trimethoxysilan getränkt und erneut getrocknet. Danach wurden die Teile evakuiert und mit einer evakuierten 1:1-Mischung aus Urethandimethacrylat und Triethylenglykoldimethacrylat über Nacht bei Raumtemperatur infiltriert und anschließend über einen Zeitraum von 17 Std. polymerisiert , wobei die Spitzentemperatur 80 °C betrug.

Die Teile zeigten auf einer Universalprüfmaschine der Fa. Zwick eine mittlere Biegefestigkeit von 256,87 MPa und einen Elastizitätsmodul von 82,89 GPa.

### Beispiel 3:

Ein bimodales Gemisch aus 67% Aluminiumoxid und 33% Zirkondioxid mit einem d-50 von ca. 2,5 µm wurde mit destilliertem Wasser und üblichen Zusätzen (hier Citronensäure und Darvan) sowie Ultraschall zu einer schlickerfähigen Suspension angerührt. Mit dieser Suspension wurden Delrinformen mit den Maßen 1,2x4x20mm ausgegossen. Nach Trocknung wurden die Teile entformt und bei einer Spitzentemperatur von 1180 °C und einer Haltezeit von 2 Stunden gebrannt.

Die gebrannten porösen Teile wurden dann mit einer 5%igen Lösung von Methacryloxypropyl-Trimethoxysilan getränkt und erneut getrocknet. Danach wurden die Teile evakuiert und mit einer evakuierten 1:1-Mischung aus Urethandimethacrylat und Triethylenglykoldimethacrylat über Nacht bei Raumtemperatur infiltriert und anschließend über einen Zeitraum von 17 Std. polymerisiert , wobei die Spitzentemperatur 80 °C betrug.

Die Teile zeigten auf einer Universalprüfmaschine der Fa. Zwick eine mittlere Biegefestigkeit von 287,42 MPa und einen Elastizitätsmodul von 79,12 GPa.

### Beispiel 4:

Ein bimodales Gemisch aus 2 Feldspatfritten (Fritte 1 Brenntemperatur ca. 830 °C, 10% Anteil an der Mischung und Fritte 2 Brenntemperatur ca. 1180 °C, 90% Anteil an der Mischung) mit einem d-50 von ca. 4,5µm wurde mit einer üblichen Modellierflüssigkeit (Wasser + Binderzusatz) zu einer schlickerfähigen Suspension angerührt. Diese Suspension wurde in Metallformen mit den Maßen 25x5x1,6 mm eingerüttelt. Nach Trocknung wurden die Teile entformt und bei einer Spitzentemperatur von 940 °C und einer Haltezeit von ca. 40 Minuten gebrannt.

Die gebrannten porösen Teile wurden dann mit einer 5%igen Lösung von Methacryloxypropyl-Trimethoxysilan getränkt und erneut getrocknet. Danach wurden die Teile evakuiert und mit einer evakuierten 1:1-Mischung aus Urethandimethacrylat und Triethylenglykoldimethacrylat über Nacht bei Raumtemperatur infiltriert und anschließend über einen Zeitraum von 17 Std. polimerisiert, wobei die Spitzentemperatur 80 °C betrug.

Die Teile zeigten auf einer Universalprüfmaschine der Fa. Zwick eine mittlere Biegefestigkeit von 148,83 MPa und einen Elastizitätsmodul von 30,04 GPa.

Die Teile zeigen eine hervorragende Transluzenz und sind auf Grund ihrer optischen Eigenschaften für ästhetische Dentalrestaurationen geeignet.

## Patentansprüche

1. Verbundwerkstoff mit einer porösen anorganisch nichtmetallischen Matrix und einem zweiten Material, **dadurch gekennzeichnet, dass**
- die poröse anorganische nichtmetallische Matrix eine Biegebruchfestigkeit von ≥ 40 Mpa, gemessen nach ISO 6 872, besitzt,
- das zweite Material ein organisches Material ist, das die Poren der porösen Matrix mindestens teilweise ausfüllt und
- der Verbundwerkstoff einen Elastizitätsmodul E ≥ 25 GPa gemessen nach ISO 10 477 aufweist.

2. Verbundwerkstoff nach Anspruch 1, **dadurch gekennzeichnet, dass** er eine Biegebruchfestigkeit von σ ≥ 100 Mpa, gemessen nach ISO 6 872 aufweist.

3. Verbundwerkstoff nach Anspruch 1 und/oder 2, **dadurch gekennzeichnet, dass** der Sinterprozess erst nach der Phase der Sinterhalsbildung und/oder des Glasflusses beendet wird.

4. Verbundwerkstoff nach mindestens einem der Ansprüche 1 bis 3, **dadurch gekennzeichnet, dass** pulverförmige anorganische Substanzen oder Substanzmischungen mit Korngrößen eingesetzt werden, die eine bimodale Verteilung aufweisen.

5. Verbundwerkstoff nach mindestens einem der Ansprüche 1 bis 4, **dadurch gekennzeichnet, dass** die pulverförmigen anorganischen. Substanzen oder Substanzmischungen eine Korngröße d₅₀ von 0,2 µm bis 25 µ, vorzugsweise von 0,5 bis 5 µm, gemessen mittels Lasergranulometrie, aufweisen.

6. Verbundwerkstoff nach mindestens einem der Ansprüche 1 bis 5, **dadurch gekennzeichnet, dass** das anorganisch- nichtmetallische Netzwerk eine mit einem Kopplungsreagenz belegt Oberfläche aufweist, die **dadurch** hydrophobiert und mit funktionellen Gruppen versehen wird.

7. Verbundwerkstoff nach mindestens einem der Ansprüche 1 bis 6, **dadurch gekennzeichnet, dass** das Silanisierungsmittel Aminopropyltriethoxysilan, Vinyltriethoxysilan, 3-M-ethacryloxypropyl-trimethoxysilan oder eine Mischung derselben ist.

8. Verfahren zur Herstellung eines Verbundwerkstoffs nach mindestens einem der Ansprüche 1 bis 7, **dadurch gekennzeichnet, dass** es folgende Schritte umfasst:
- Herstellung des anorganischen Ausgangsmaterials, vorzugsweise in Pulverform,
- Sintern des anorganischen Ausgangsmaterials, wobei der Sinterprozess beendet wird, bevor im wesentlichen geschlossene Poren im Sinterprodukt entstehen,
- und die Phase der Sinterhalsbildung und/oder des Glasflusses erreicht worden ist,
- die Oberfläche der porösen anorganisch nichtmetallischen Matrix mit einem Kopplungsreagenz belegt wird,
- die so erhaltene poröse und oberflächenmodifizierte anorganische nichtmetallische Matrix mit organischem Material infiltriert wird und
- das organische Material danach verfestigt wird.

9. Verfahren nach Anspruch 8, **dadurch gekennzeichnet, dass** organische Monomere oder Präpolymere in das gesinterte anorganische Material verbracht werden und in den Poren des gesinterten anorganischen Materials zu dem organischen Material polymerisiert werden.

10. Verfahren nach Anspruch 9, **dadurch gekennzeichnet, dass** die Polymerisation unter Druck durchgeführt wird.

11. Verfahren nach Anspruch 9 und/oder 10, **dadurch gekennzeichnet, dass** als Monomere organische Verbindungen eingesetzt werden, die mindestens eine ethylenisch ungesättigte Struktureinheit, mindestens eine kondensierbare Struktureinheit und/oder mindestens eine ringöffnend polymerisierbare Struktureinheit enthalten.

12. Verfahren nach Anspruch 9 bis 11, **dadurch gekennzeichnet, dass** radikalisch polymerisierbare Monomere, wie Acrylate, Methacrylate, zusammen mit Radikalbildnern als Starter, wie Azoverbindungen oder Peroxiden, eingesetzt werden.

13. Verfahren nach mindestens einem der Ansprüche 8 bis 12, **dadurch gekennzeichnet, dass** feldspathaltige Pulver und Fritten als anorganisches Material und Bismethacrylate als organische Verbindung sowie peroxidhaltige Verbindungen als Starter eingesetzt werden.

14. Verwendung eines Verbundwerkstoffs nach mindestens einem der Ansprüche 1 bis 7 für dentale Zwecke, z.B. für Inlays, Onlays, Kronen und Brücken.

15. Verwendung eines Verbundwerkstoffs nach mindestens einem der Ansprüche 1 bis 7 als Gleitlager, als Wärme- und/oder Schalldämmung, Vibrationsdämpfer, sowie zur Oberflächenvergütung von Metallen, Kompositen und Keramiken.

16. Mehrschichtiger Formkörper aus einem Verbundwerkstoff nach einem des Ansprüche 1 bis 7 mit mehreren Schichten mit unterschiedlichen Eigenschaften in den Schichten erhältlich durch Brennen einer homogenen Matrix in einem Gradientenofen.

17. Verfahren zur Herstellung eines Formkörpers mit mehreren Schichten mit unterschiedlichen Eigenschaften in den Schichten nach Anspruch 16, wobei ein Aufbau des Formkörpers mittels Härtung einzelner Schichten und Auftrag neuer Schichten mit anderen Ausgangsmaterialien mit anderen physikalisch-technischen Eigenschaften erfolgt und eine homogene Matrix aus einem porösen anorganischen nichtmetallischen Material in einem Gradientenofen gebrannt wird.

## Claims

1. A composite material with a porous inorganic-nonmetallic matrix and a second material, **characterized in that**
- said porous inorganic-nonmetallic matrix has a bending strength of ≥ 40 MPa as measured according to ISO 6 872;
- said second material is an organic material which at least partly fills the pores of said porous matrix; and
- said composite material has a modulus of elasticity, E, of ≥ 25 GPa as measured according to ISO 10 477.

2. The composite material according to claim 1, **characterized by** having a bending strength, σ, of ≥ 100 MPa as measured according to ISO 6 872.

3. The composite material according to claim 1 and/or 2, **characterized in that** the sintering process is discontinued only after the phase of necking and/or glass flow.

4. The composite material according to at least one of claims 1 to 3, **characterized in that** powdery inorganic substances or mixtures of substances having grain sizes which exhibit a bimodal distribution are employed.

5. The composite material according to at least one of claims 1 to 4, **characterized in that** said powdery inorganic substances or mixtures of substances have a grain size d₅₀ of from 0.2 µm to 25 µm, preferably from 0.5 to 5 µm, as measured by laser granulometry.

6. The composite material according to at least one of claims 1 to 5, **characterized in that** the inorganic-nonmetallic network has a surface coated with a coupling agent which is thus hydrophobized and provided with functional groups.

7. The composite material according to at least one of claims 1 to 6, **characterized in that** the silanizing agent is aminopropyltriethoxysilane, vinyltriethoxysilane, 3-methacryloxypropyltrimethoxysilane or a mixture thereof.

8. A method for the preparation of a composite material according to at least one of claims 1 to 7, **characterized by** comprising the following steps:
- preparing the inorganic starting material, preferably in a powdery form;
- sintering the inorganic starting material, the sintering process being discontinued before substantially closed pores are formed in the sintered product;
- and before the phase of necking and/or glass flow has been reached;
- the surface of the porous inorganic-nonmetallic matrix is coated with a coupling agent;
- the thus obtained porous and surface-modified inorganic-nonmetallic matrix is infiltrated with an organic material; and
- the organic material is subsequently solidified.

9. The method according to claim 8, **characterized in that** organic monomers or prepolymers are introduced into the sintered inorganic material and polymerized within the pores of the sintered inorganic material to form the organic material.

10. The method according to claim 9, **characterized in that** said polymerization is effected under pressure.

11. The method according to claim 9 and/or 10, **characterized in that** organic compounds which contain at least one ethylenically unsaturated moiety, at least one condensable moiety and/or at least one moiety capable of ring-opening polymerization are employed as the monomers.

12. The method according to claim 9 to 11, **characterized in that f**ree-radical polymerizable monomers, such as acrylates, methacrylates, are employed together with free-radical formers as initiators, such as azo compounds or peroxides.

13. The method according to at least one of claims 8 to 12, **characterized in that** feldspar-containing powders and frits are employed as the inorganic material, and bismethacrylates are employed as the organic compound, and peroxide-containing compounds are employed as initiators.

14. Use of a composite material according to at least one of claims 1 to 7 for dental purposes, such as for inlays, onlays, crowns and bridges.

15. Use of a composite material according to at least one of claims 1 to 7 as a friction bearing, for heat and/or sound insulation, as a vibration damper, and for the surface coating of metals, composites and ceramics.

16. A multilayered molded part made from a composite material according to any of claims 1 to 7 and having several layers with different properties in the layers, obtainable by firing a homogeneous matrix in a gradient oven.

17. A method for the preparation of a molded part having several layers with different properties in the layers according to claim 16, wherein the molded part is assembled by curing individual layers and applying new layers with different starting materials having different physical-technical properties and a homogeneous matrix of a porous inorganic-nonmetallic material is fired in a gradient oven.

## Revendications

1. Matériau composite comportant une matrice minérale non métallique poreuse et une deuxième matière, **caractérisé en ce que**
- la matrice minérale non métallique poreuse présente une résistance au pliage à la rupture de ≥ 40 Mpa, mesurée conformément à ISO 6 872,
- la deuxième matière est un matériau organique qui remplit au moins partiellement les pores de la matrice poreuse et
- le matériau composite a un module d'élasticité de E ≥ 25 GPa mesuré conformément à ISO 10 477.

2. Matériau composite selon la revendication 1, **caractérisé en ce qu'**il présente une résistance au pliage à la rupture de σ ≥ 100 Mpa, mesurée conformément à ISO 6 872.

3. Matériau composite selon la revendication 1 et/ou 2, **caractérisé en ce que** le processus de frittage n'est terminé qu'après la phase de formation de ponts par frittage et/ou de flux de gaz.

4. Matériau composite selon au moins l'une des revendications 1 à 3, **caractérisé en ce que** l'on utilise des substances ou des mélanges de substances minérales pulvérulentes dont la granulométrie présente une distribution bimodale.

5. Matériau composite selon au moins l'une des revendications 1 à 4, **caractérisé en ce que** les substances ou mélanges de substances minérales pulvérulentes ont une granulométrie d₅₀ de 0,2 µm à 25 µm, de préférence de 0,5 à 5 µm, mesurée par analyse granulométrique par laser.

6. Matériau composite selon au moins l'une des revendications 1 à 5, **caractérisé en ce que** le réseau minéral non métallique présente une surface qui est recouverte d'un réactif de couplage, qui est ainsi rendue hydrophobe et qui est dotée de groupes fonctionnels.

7. Matériau composite selon au moins l'une des revendications 1 à 6, **caractérisé en ce que** l'agent de silanisation est l'aminopropyltriéthoxysilane, le vinyltriéthoxysilane, le 3-méthacryloxypropyltriméthoxysilane ou un mélange de ceux-ci.

8. Procédé de fabrication d'un matériau composite selon au moins l'une des revendications 1 à 7, **caractérisé en ce qu'**il comporte les étapes suivantes :
- la fabrication du matériau minéral de départ, de préférence sous forme pulvérulente,
- le frittage du matériau minéral de départ, le processus de frittage étant terminé avant l'apparition des pores sensiblement fermés dans le produit fritté,
- et **en ce que**, une fois que la phase de formation de ponts par frittage et de flux de gaz a été atteinte,
- la surface de la matrice minérale non métallique poreuse est recouverte d'un réactif de couplage,
- la matrice minérale non métallique poreuse à surface modifiée qui est ainsi obtenue est infiltrée par un matériau organique et
- puis le matériau organique est solidifié.

9. Procédé selon la revendication 8, **caractérisé en ce que** des monomères ou prépolymères organiques sont introduits dans le matériau minéral fritté et sont polymérisés dans les pores du matériau minéral fritté pour donner le matériau organique.

10. Procédé selon la revendication 9, **caractérisé en ce que** la polymérisation est réalisée sous pression.

11. Procédé selon la revendication 9 et/ou 10, **caractérisé en ce que** les monomères utilisés sont des composés organiques qui contiennent au moins une unité structurellement éthylèniquement insaturée, au moins une unité structurelle condensable et/ou au moins une unité structurelle polymérisable avec ouverture de cycles.

12. Procédé selon les revendications 9 à 11, **caractérisé en ce que** des monomères radicalement polymérisables, tels que des acrylates, des méthacrylates, sont utilisés conjointement avec des générateurs de radicaux servant d'amorces, tels que des composés azoïques ou des peroxydes.

13. Procédé selon au moins l'une des revendications 8 à 12, **caractérisé en ce que** des frittes et des poudres contenant du feldspath sont utilisées comme matériau minéral, des bisméthacrylates sont utilisés comme composé organique et des composés contenant des peroxydes sont utilisés comme amorce.

14. Utilisation de matériaux composites selon l'une des revendications 1 à 7 en dentisterie, par exemple pour des Inlays, Onlays, couronnes et bridges.

15. Utilisation d'un matériau composite selon l'une des revendications 1 à 7 pour des paliers lisses, pour l'isolation thermique et/ou acoustique, pour des amortisseurs de vibration, ainsi que pour le traitement de surface de métaux, de composites et de céramiques.

16. Corps profilé multicouches constitué d'un matériau composite selon l'une des revendications 1 à 7, ledit corps comportant plusieurs couches qui présentent différentes propriétés et étant obtenu par cuisson d'une matrice homogène dans un four à gradient.

17. Procédé de fabrication d'un corps profilé multicouches dont les couches présentes différentes propriétés selon la revendication 16, le corps profilé étant réalisé par durcissement de couches individuelles et par application de nouvelles couches constituées d'autres matériaux de départ présentant de d'autres propriétés physico-chimiques et une matrice homogène constituée d'un matériau minéral non métallique poreux étant cuit dans un four à gradient.
